Europäisches Patentamt

European Patent Office

Office européen des brevets

(11) Publication number: **0 562 125 A1**

# EUROPEAN PATENT APPLICATION
## published in accordance with Art. 158(3) EPC

(21) Application number: 93906345.9

(22) Date of filing: **09.10.92**

(86) International application number:
**PCT/JP92/01316**

(87) International publication number:
**WO 93/06862 (15.04.93 93/10)**

(51) Int. Cl.5: **A61K 39/395**, A61K 9/72

(30) Priority: **11.10.91 JP 263926/91**

(43) Date of publication of application:
**29.09.93 Bulletin 93/39**

(84) Designated Contracting States:
**DE FR GB IT**

(71) Applicant: **TORAY INDUSTRIES, INC.
2-1, Nihonbashi Muromachi 2-chome
Chuo-ku
Tokyo 103(JP)**

(72) Inventor: **YAMAZAKI, Shojiro B-2, 2-24,
Tsunishi 2-chome
Kamakura-shi
Kanagawa-ken 248(JP)**
Inventor: **SONE, Saburo 1120-3, Yoshida-cho
Totsuka-ku
Yokohama-shi
Kanagawa-ken 244(JP)**
Inventor: **KAJITA, Akemi
936-16, Daigiri Fujisawa-shi
Kanagawa-ken 251(JP)**

(74) Representative: **Kador & Partner
Corneliusstrasse 15
D-80469 München (DE)**

(54) **ANTIBODY COMPOSITION.**

(57) An antibody composition prepared by lyophilizing an antibody or a complex thereof and finely pulverizing the product of lyophilization and having an effective particle diameter of 0.5 μm to less than 10 μm. Since this composition has an excellent stability, it is suitable as a transpulmonary composition of an antibody or a complex thereof useful as a medicine.

# F i g. 1

Field of the Invention

The present invention relates to a novel antibody containing medicine which is pharmaceutically useful. More particularly, it relates to a composition containing the antibody or its complex such as an immunotoxin complex and the like.

Background of the Invention

Recently, an application of the antibodies to the medicines is extensively studied. The research and development of pharmaceutical formulations containing the antibodies in various fields including the fields of tumor, cardiac and circulatory system, immunity, allergy and infection have been advanced extensively. For example, the research for treating cancer with monoclonal antibodies against tumor-associated antigens is most active. Such antibodies will be developed as the pharmaceutical formulations in the near future. At present, some formulations are subjected to clinical experiments wherein the tumor sites to be treated are covered with a wide range, including blood, hematopoietic organ, lung, liver, digestive organ, ovarium and prostate. As a method for treating the cancer with the monoclonal antibody, an immunotherapy with only the antibody so that macrophagus and lymphocytes having receptors against Fc fragments of the antibody are collected at the tumor sites, thereby the cancer is attacked and a target therapy with the immunotoxin complex comprising the monoclonal antibody to which a toxic substance such as a toxic protein, a radioactive substance (RI) and an anticancer agent is bound are known.

Dosage forms for administrating the monoclonal antibodies are all injections, because the monoclonal antibodies are macromolecular protein. Most of the injections are the injections for intravenous administration. Because the injection is a troublesome and imposes a burden to a patient, simpler dosage form is desired. One is an inhalation. By the inhalation having a dosage form for general administration, the burden to the patient can be lightened and the patient can be treated at home. On the other hand, when the immunotoxin complex wherein the monoclonal antibody is bound with, for example, the toxic protein is intravenously administered, it circulates through major organs including liver and kidney and as the result, these organs may be seriously damaged. Thus, the dosage form in place of the injection is desired. One is the inhalation for a topical administration in order to treat lung cancer. It is expected to be effectively used. However, the inhalation has a defect because of a drug used being macromolecular.

One of the inhalations is an aqueous inhalation, wherein the drug in the form of water-soluble microparticles is administered using a suitable device such as a jet nebulizer and an ultrasonic nebulizer. The other is a solid inhalation, wherein the drug in the form of solid particulate powder is administered using a metered-dose inhaler. The drugs in both inhalations are mainly low molecular compounds such as $\beta_2$-adrenergic antagonists, steroids and antibiotics.

An attempt for formulating polypeptides as aerosol preparations is relatively new. For example, a solid aerosol preparation containing insulin (Lee and Sciarra, J. Pharm., 65, 567, 1976), a solid aerosol preparation containing recombinant $\alpha_1$-antitrypsin (Hubbard et al., Proc. Natl. Acad. Sci. USA, 86, 680, 1989), the administration of an aqueous solution of human leukocyte interferon-$\alpha$ through the jet nebulizer (Kin-nula et al., J. Interferon Res., 9, 419, 1989) and the administration of an aqueous solution of human growth hormone through the nebulizer (JP-A-63-51868) are reported. They describe that the polypeptide having the molecular weight of 50,000 or less is used. And, they describe that the preferable particle diameter is 0.5 to 10 $\mu$m, particularly 5 $\mu$m or less, for adsorbing the polypeptides by the inhalation. The effectiveness of certain drugs as the dosage form for the topical administration has been also examined.

In case of the aerosol preparation, it is considered that the absorption sites are determined depending on the particle size. It is said that smaller particles can reach to alveoli pulmonum, while larger particles deposit in a nasal or oral cavity. For reaching to the alveoli pulmonum, the particle diameter should be 0.5 to 10 $\mu$m (Porush et al., J. Amer. Pharm. Ass. Sci., Ed., 49, 70, 1960), preferably 5 $\mu$m or less in diameter (Newman and Clarks, Thorax, 38, 881, 1983).

At present, the inhalation of the antibody is pharmaceutically desired as a simple general administration and as a topical administration to the alveoli pulmonum. However, the macromolecular substance such as the monoclonal antibody having the molecular weight of 150,000 among the polypeptides is hardly adsorbed through the inhalation. Nevertheless, the absorption of the macromolecular substance through the inhalation and the retention thereof in the lung through the topical administration are important and strongly desired. Unfortunately, the technic for obtaining the stable preparation containing the antibody while maintaining its physiological activities is not well established.

The monoclonal antibody having the physiological activities such as a binding capacity to cancer cells is easily affected in a solution system, with respect to its stability and pharmaceutical activities. For example, it is frequently deactivated by oxidation, coagulation and thermal denaturation in the solution system. When its aqueous solution is nebulized through the jet nebulizer or the ultrasonic nebulizer, a shear stress is applied to the monoclonal antibody. Therefore, the powdery preparation is preferable and excellent. Irrespective of the form (aqueous or solid) of the preparation, the selections of a stabilizer and stabilizing conditions are essential in order to obtain the stable preparation containing the monoclonal antibody which has higher pharmaceutical activity.

An object of the present invention is to obtain an antibody containing composition which is expected to have a pharmaceutical applicability, as a stable solid aerosol preparation capable of being inhaled.

## Summary of the Invention

The above object will be attained by the present invention. The present invention relates to a composition containing an antibody or its complex, which is obtained by freeze-drying a solution of an antibody or its complex followed by milling and which has an effective particle diameter of more than 0.5 $\mu$m and less than 10 $\mu$m.

## Brief Explanation of Drawings

Fig. 1 shows a serum concentration after inhaling to rabbits each of the MDI preparations a) and c) which were prepared in Example 1, as a function of time. - ○ - is the serum concentration of human Fab in the rabbits to which the MDI preparations a) were administered, and - ● - is that of human IgG in the rabbits to which the the MDI preparations c) were administered.

Fig. 2 shows a serum concentration after inhaling to the rabbits the MDI preparation e) which was prepared in Example 2, as a function of time.

## Best Mode for carrying out the Invention

The antibody herein includes immunoglobulins (IgGs), their fragments or their functionally equivalent molecules, and their products modified by a genetic engineering technic such as a chimera antibody in which constant regions in an amino acid sequence are replaced with other amino acid sequences. As the antibody fragments, F(ab')2, Fab', Fab and Fv which are produced by conventional methods are exemplified. The antibody usable in the present invention is not limited to either polyclonal or monoclonal antibody. And, an animal species from which the antibody is derived is not limited.

The antibody having the physiologically activities such as the binding capacity to the cancer cells can be applied, for example, to the cancer treatment. When the antibody is systemicaly administered for treating the cancer, the antibody used is not limited. When the antibody is topically administered to lung, however, only the antibody specific to the pulmonary cancer is used. For example, SF25 [Cancer Res., 48, 6573-6579 (1988)], XF8, AF20 [Hepatology, 9, 625-634 (1989)], SWA11 [Br. J. Cancer, 59, 174-178 (1989)], SM1 [Cancer Res., 44, 265 (1984)] and TFS-4 [Cancer Res., 47, 826 (1987)] can be used. As the method for treating the cancer, the immunotherapy is mentioned, in which the antibody is singly administered and thereby, the macrophagus or the lymphocytes which have the receptors against Fc regions of the antibody are collected on the tumor sites so as to attack the tumor. Alternatively, the target therapy is mentioned, in which the complex comprising the antibody to which the toxic substance (for example, the toxic protein, the radioactive substance (RI) and anticancer agent) is bound is used. In the complex comprising the antibody and the toxic substance, the toxic protein as one of the usable toxic substances includes A chains of plant toxins (type II) such as ricin and abrin and active components of plant toxins (type I) such as luffin, momordin and PAP-S and of bacterial protein toxin such as Pseudomonas toxin and diphtheria toxin, which irreversibly terminates a protein synthesis and thereby a high toxicity is given to the cells. The other preferable toxic protein includes an enzyme derived from human such as human RNase, which is incorporated into the cells and thereby lethal damages can be given to the cells. In the preparation of the immunotoxin complex by binding the antibody with the toxic protein, a crosslinking agent is used [Tanpakushitsu-Kakusan-Koso (PROTEIN NUCLEIC ACID ENZYME), supplement No. 31, p.335-343 (1987)].

For stabilizing the antibody, the addition of a sugar such as lactose, maltose, sorbitose, tolehalose and xylose and/or a sugar alcohol such as mannitol, sorbitol and xylitol is effective in the present invention. The addition of the protein such as human serum albumin is also effective for stabilizing the antibody. The added amount of the sugar or sugar alcohol is preferably 0.01 to 200 %, preferably 1 to 50 % based on the total weight of the antibody and the human serum albumin.

4

The milling of the freeze-dried product is conducted using a suitable apparatus such as a jet milling apparatus (for example "Micronizer Mill") and a ball milling apparatus. For the inhalation, the particles have a particle size distribution such that more than 50 % or more, preferably 75 % or more of the total particles have the particle diameter of 0.5 to 5 $\mu$m. The particle size distribution can be determined by a standard particle size distribution analyzer (for example, model CAPA-700 of Horiba Ltd. and QCM-Cascade Impactor model PC-2 of California Measurement Inc.).

It is preferable to add any surfactant to the freeze-dried and milled product for improving the dispersibility of the particles. The surfactant used in the present invention is at least one of sorbitan trioleate (Span 85), oleyl alcohol, soya lecitin and hydrogenated castor oil derivatives (HCO60). The addition amount of the surfactant is 0.001 to 5 %, preferably 0.05 to 2 % based on the total weight of the antibody and the human serum albumin.

Optionally, mineral ions which are necessary to keep an osmotic pressure intrinsically present in an organism, such as calcium, magnesium, sodium, potassium, chloride and phosphate ions may be added. If necessary, a surfactant derived from alveoli pulmonum may be added for controlling a stimulating property to the organism.

The thus-prepared antibody containing composition is as such or dispersed in a nebulizing gas such as compressed air and compressed carbon oxide gas or in a suitable propellant in order to inhale through the oral or nasal cavity. The usable propellant includes chlorofluorocarbon (furon 11, 12, 114 etc.), hydrochlorofluorocarbon (furon 123, 124, 141b) and fluorocarbon (furon 125, 134a).

Examples

The following examples will more fully illustrate the present invention, but they are not intended to be limiting of the present invention.

Example 1

Powders were prepared by freeze-drying 20 ml of a solution containing 0.75 mg/ml of a freeze-dried powder of human immunoglobulin IgG (ex. PAESEL) or human immunoglobulin Fab (ex. CAPPEL), 15.0 mg/ml of human serum albumin, 2.0 mg/ml of sorbitol, 0.375 mg/ml of phosphate buffer and 1.5 mg/ml of sodium chloride. Then, each of the freeze-dried solid powders was collected followed by milling in Sturtevant jet milling apparatus (500 ml capacity), thereby a milled powder was obtained. The resultant milled powder was analyzed using the particle size distribution analyzer (model CAPA-700 of Horiba Ltd.). The milled powder comprising IgG had the average particle diameter of 5.20 ± 1.86 $\mu$m, the proportion of the particles with the particle diameter of 5.45 $\mu$m or less being 55.0 %. While, the milled powder comprising Fab had the average particle diameter of 1.95 ± 1.05 $\mu$m, the proportion of the particles with the particle diameter of 4.00 $\mu$m or less being 97.9 %.

Next, 50 to 100 mg of the powder was placed in a glass vial (10 ml capacity), to which oleyl alcohol or sorbitan trioleate (Span 85) was added such that it is contained in an amount of 0.25 % under nitrogen atmosphere. After a metered-dose valve was attached on the glass vial, furon 12 was introduced therein so that the total amount was 10 ml. Thus, four MDI (metered - dose inhalation) preparations a), b), c) and d) were obtained. A composition of each preparation was shown in Table 1.

Table 1

MDI preparations comprising human immunoglobulin IgG and human immunoglobulin Fab'

| composition and dose | | | | |
|---|---|---|---|---|
| MDI preparation | composition | amount (w/v %) | weight (g) | dose (μg/time) |
| a) Fab (oleyl alcohol) | Fab powder* oleyl alcohol CFC 12 | 0.5 0.25 99.25 | 0.050 0.025 13.27 | 9.55 |
| b) Fab (SPAN 85) | Fab powder* SPAN 85 CFC 12 | 1.0 0.25 98.75 | 0.10 0.025 13.20 | 19.1 |
| c) IgG (oleyl alcohol) | IgG oleyl alcohol CFC 12 | 1.0 0.25 98.25 | 0.10 0.025 13.20 | 500 |
| d) IgG (SPAN 85) | IgG SPAN 85 CFC 12 | 1.0 0.25 98.25 | 0.10 0.025 13.20 | 500 |

* Fab powder contained the following ingredients per 100 mg:

Fab powder : 3.82 mg

HSA : 76.4 mg

phosphate : 1.91 mg

NaCl : 7.6 mg

D-sorbitol : 10.2 mg

Using a metered-dose inhaler, the human immunoglobulin Fab or IgG in a dose shown in Table 1 was inhaled to rabbits. Three NZW male rabbits of body weight of 3.0 to 3.5 kg were used per each group. Before the administration to the rabbit, the MDI preparation was forcedly sprayed in a bellows-type spacer five times. Then, an adapter was attached, through which the MDI preparation was administered to the lung of the rabbit by a spontaneous inhalation for one minute. For each of the MDI preparations c), d), a) and b), the above procedure was repeated two times, four times, six times and six times, respectively. Accordingly, the expected final dose of the immunoglobulin was 5 mg, 10 mg, 287 μg and 573 μg, respectively.

After 0.5, 1, 3, 6, 9 and 12 hours from the administration of the MDI preparation, a blood was sampled from an ear auricular vein of each rabbit. A serum in a blood sample was separated so as to prepare a sample for determining a serum concentration of human immunoglobulin IgG or Fab. The determination is conducted according to the EIA method. Firstly, an immunoplate (ex. NUNC) was coated with 100 μl of a 10 μg/ml solution of a sheep anti-human F(ab')₂ antibody (ex. CAPPEL) in PBS(-) at 4°C overnight. After washing with 250 μl of PBS(-), the plate was blocked with 1% BSA/PBS(-) at 4°C overnight followed by washing with each of 250 μl of a washing solution (0.05% Tween 20/PBS(-)) three times. Then, a human immunoglobulin IgG standard (ex. ZYMED), a human immunoglobulin Fab standard (ex. CAPPEL) and a

EP 0 562 125 A1

sample were added and the reaction was continued at room temperature for 1 hour. After washing with each of 250 $\mu$l of the washing solution three times, 100 $\mu$l of a horseraddish peroxidase (HRP) standard goat anti-human IgG (H + L) chains antibody (ex. ZYMED) which was previously diluted 12,000 times was reacted at room temperature for 1 hour. Thereafter, the washing was repeated. A substrate for HRP was added in order to develop a color. After the reaction was terminated, an absorption at 490 nm was detected using an immuno reader.

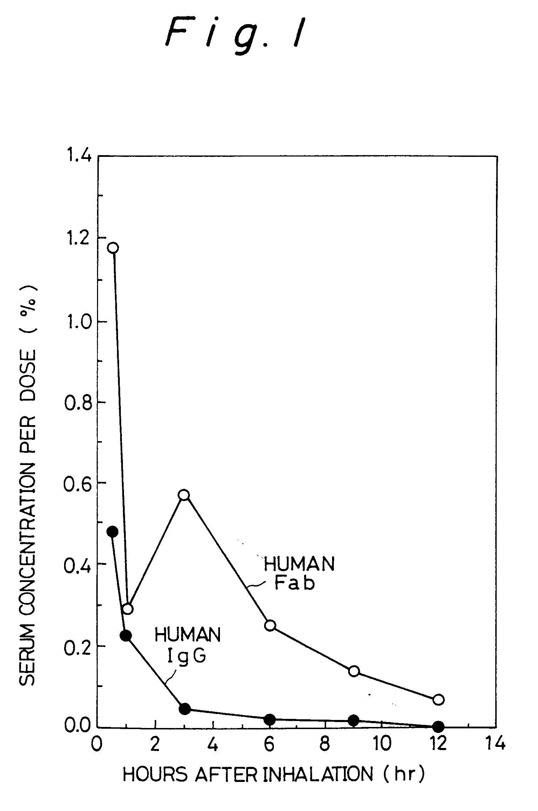

The results showed that in both preparations comprising the human immunoglobulins IgG and Fab, the preparations comprising oleyl alcohol showed higher absorption efficiency, as compared with those comprising SPAN 85. Thus, the change in serum concentration after the inhalation of the preparation (a) or (c) comprising oleyl alcohol was determined. The results are shown in Fig. 1. After 30 minutes from the inhalation, the serum concentration was reached to a maximum value in both preparations a) and c) . In the preparation a), it was 1.18 % of the dose. In the preparation c), it was 0.48 % of the dose. Further, Fab showed the higher serum concentration as compared with IgG.

Example 2

A IgG-Abrin A chain complex as the immunotoxin complex was prepared from human immunoglobulin IgG (ex. PAESEL) and abrin A chain using SPDP (N-succinimidyl 3-(2-pyridyl-dithio)propionate) of the crosslinking agent. For the purification of the complex, blue Sepharose column and a gel filtration column were used.

A powder was prepared by freeze-drying 20 ml of a solution containing 0.7 mg/ml of IgG-abrin A chain complex, 15.0 mg/ml of human serum albumin, 2.0 mg/ml of sorbitol, 0.75 mg/ml of phosphate buffer and 1.5 mg/ml of sodium chloride. The freeze-dried powder was milled in Sturtevant jet milling apparatus (500 ml capacity), thereby a milled powder was obtained. The resultant milled powder was analyzed using the particle size distribution analyzer (model CAPA-700 of Horiba Ltd.). The milled powder had the average particle diameter of 4.65 ± 1.58 $\mu$m, the proportion of the particles with the particle diameter of 5.00 $\mu$m or less being 63.6 %.

One hundred mg of the powder was placed in a glass vial (10 ml capacity), to which oleyl alcohol was added such that it is contained in an amount of 0.25 % under nitrogen atmosphere. After a metered-dose valve was attached on the glass vial, furon 12 was introduced therein so that the total amount was 10 ml. Thus, MDI preparations e) was obtained.

Using the metered-dose inhaler, the complex in a dose of 500 mg/time was inhaled to three NZW male rabbits of body weight of 3.0 to 3.5 kg per each group. Before the administration to the rabbit, the MDI preparation was forcedly sprayed in a bellows-type spacer five times. Then, an adapter was attached, through which the MDI preparation was administered to the lung of the rabbit by a spontaneous inhalation for one minute. The above procedure was repeated two times. Accordingly, the expected final dose of the complex was 5 mg.

After 0.5, 1, 3, 6, 9 and 12 hours from the administration of the MDI preparation, a blood was sampled from an ear auricular vein of each rabbit. A serum in a blood sample was separated so as to prepare a sample for determining a serum concentration of the complex. The determination is conducted according to the EIA method.

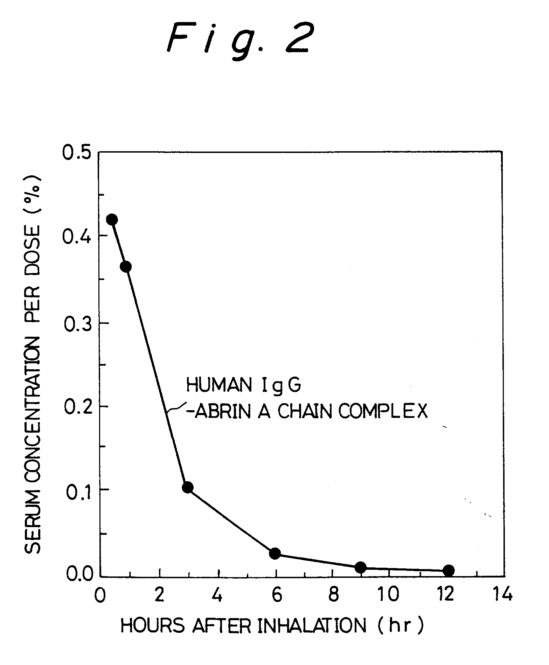

The change in serum concentration after the inhalation is shown in Fig. 2. After 30 minutes from the inhalation, the serum concentration was reached to a maximum value. It was confirmed to be 0.42 % of the dose.

Utilizability in Industries

Because the antibody containing composition of the present invention is the stable solid composition, it is suitable as the composition for the inhalation of the antibody or its complex which is pharmaceutically effective.

**Claims**

**1.** A composition containing an antibody or its complex, which is obtained by freeze-drying a solution of the antibody or its complex followed by milling the freeze-dried product so that it has an effective particle diameter of more than 0.5 $\mu$m and less than 10 $\mu$m.

**2.** The composition as claimed in claim 1, which is a composition for inhalation.

7

# F i g. I

# Fig. 2

# INTERNATIONAL SEARCH REPORT

## I. CLASSIFICATION OF SUBJECT MATTER (If several classification symbols apply, indicate all) [6]

According to International Patent Classification (IPC) or to both National Classification and IPC

Int. Cl$^5$   A61K39/395, A61K9/72

## II. FIELDS SEARCHED

### Minimum Documentation Searched [7]

| Classification System | Classification Symbols |
|---|---|
| IPC | A61K39/395, A61K9/72 |

Documentation Searched other than Minimum Documentation
to the Extent that such Documents are included in the Fields Searched [8]

## III. DOCUMENTS CONSIDERED TO BE RELEVANT [9]

| Category [*] | Citation of Document, [11] with indication, where appropriate, of the relevant passages [12] | Relevant to Claim No. [13] |
|---|---|---|
| Y | JP, A, 54-84025 (The Green Cross Corp.), July 4, 1979 (04. 07. 79), (Family: none) | 1-2 |
| Y | JP, A, 56-53622 (Machida Pharmaceutical Co., Ltd.), May 13, 1981 (13. 05. 81), (Family: none) | 1-2 |
| Y | J. Amer. Pharm. Ass. Sci. Ed., 49, 70, 1960, Porush | 1-2 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search | Date of Mailing of this International Search Report |
|---|---|
| November 26, 1992 (26. 11. 92) | January 7, 1993 (07. 01. 93) |

| International Searching Authority | Signature of Authorized Officer |
|---|---|
| Japanese Patent Office | |

Form PCT/ISA/210 (second sheet) (January 1985)